(19) **Europäisches Patentamt / European Patent Office / Office européen des brevets**

(11) **EP 1 647 227 B1**

(12) **EUROPEAN PATENT SPECIFICATION**

(45) Date of publication and mention
of the grant of the patent:
**13.08.2008 Bulletin 2008/33**

(51) Int Cl.:
**A61B 5/22** *(2006.01)*    **A61B 5/024** *(2006.01)*

(21) Application number: **05256394.7**

(22) Date of filing: **14.10.2005**

(54) **Method and apparatus for portably measuring calorie consumption.**

Verfahren und tragbarer Apparat zur Messung des Kalorieverbrauchs.

Méthode et appareil portable pour la mesure de la consommation de calories.

(84) Designated Contracting States:
**DE FR GB**

(30) Priority: **14.10.2004 KR 2004082074**
**01.04.2005 US 95551**

(43) Date of publication of application:
**19.04.2006 Bulletin 2006/16**

(73) Proprietor: **Samsung Electronics Co., Ltd.**
**Suwon-si, Gyeonggi-Do (KR)**

(72) Inventors:
• **Lee, Mihee**
**Yongin-si, Gyeonggi-do (KR)**

• **Bang, Seokwon,**
**105-1008 Saemteo Maeul Apt.**
**Seoul (KR)**
• **Lee, Hyoungki,**
**401-1902 Cheongmyeong Maeul**
**Suwon-si, Gyeonggi-do (KR)**

(74) Representative: **Greene, Simon Kenneth**
**Elkington and Fife LLP**
**Prospect House**
**8 Pembroke Road**
**Sevenoaks,**
**Kent TN13 1XR (GB)**

(56) References cited:
**WO-A-20/04109575        US-A- 5 918 603**
**US-A1- 2002 019 585**

**Description**

The invention relates to a method and apparatus for measuring heart rate, and more particularly, to a method and apparatus that can calculate calorie usage using the heart rate measured at discrete times.

In general, there are two basic techniques for measuring calorie usage. The first method is a direct method in which the heat given off by the body is directly measured. The second method is an indirect method in which the heart rate is used to estimate the calorie usage as discussed in Japanese Patent Publication No. 02-080029. However, when measuring calorie consumption using the indirect method, there generally is a need for separate machinery and/or apparatus, which makes the measurement impractical when the calorie consumption is being measured by a portable device or a general device not dedicated to measuring calorie consumption. In addition, the indirect method discussed in Japanese Patent Publication No. 02-080029 generally requires continuous measurement of the heart rate during exercise, which is often not practical or desirable for general or portable devices having more limited memory.

US 2002/0019585 A1 discloses an apparatus that calculates calories consumed during a time period based on a user's physiological characteristics, and a heart rate measured at the end of the time period. Where the heart rate is measured repeatedly, a running total of the calories expended in discrete time periods may be maintained.

According to an aspect of the invention, there is provided a measuring device for use in measuring calories consumed by a user, comprising: a pulse input unit arranged to detect a first heart rate of the user at a first point in an exercise period and a second heart rate of the user at a second point in the exercise period other than the first point, the pulse input unit not detecting a heart rate between the first and second points; and a controller arranged to receive the detected first and second heart rates and calculates calories consumed between the first and second points in the exercise period using the detected second heart rate, a predetermined at rest heart rate of the user, and one or more factors selected from an age of the user, a gender of the user, a weight of the user, a height of the user, and a percent of the body weight attributed to muscle of the user, characterized in that the controller is arranged to calculate the calories consumed between the first and second points in the exercise period further using the detected first heart rate.

According to an aspect of the invention, the controller is arranged to calculate different rates of change of the heart rate during the exercise period based upon the detected first and second heart rates.

According to an aspect of the invention, the controller calculates: a first calorie consumption component having a first rate of change during a first time period substantially at a beginning of the exercise period and during which time the user's heart rate is changing from the detected first heart rate to the detected second heart rate, and a second calorie consumption component having a second rate of change at a second time period after the first time period and during which time the user's heart rate is substantially the detected second heart rate.

According to an aspect of the invention, the first time period is ten percent of the exercise period and the second time period is ninety percent of the exercise period.

According to an aspect of the invention the controller calculates a first calorie consumption rate using the detected first heart rate, and a second calorie consumption rate using the detected second heart rate, and the controller integrates a difference between the calculated first and second calorie consumption rates over a warm up time during which the heart rate is not constant in order to calculate calories consumed during the warm up time of the exercise period.

According to an aspect of the invention, the controller calculates a rate of change of the calorie consumption rate during the warm up time to be increasing at a substantially constant rate.

According to an aspect of the invention, the controller calculates the rate of change to be substantially a difference between the calculated first and second calorie consumption rates divided by the warm up time.

According to an aspect of the invention, the controller calculates a second calorie consumption rate using the detected second heart rate, the controller calculates a regular calorie consumption rate using an at rest heart rate, the at rest heart rate being a heart rate substantially at a time when the user first awakes from sleep, and the controller integrates a difference between the calculated second and regular calorie consumption rates over a steady portion of the exercise period during which the heart rate is constant and after a warm up time during which the heart rate is not constant.

According to an aspect of the invention, a rate of change of the calorie consumption rate during the steady portion is substantially zero.

According to an aspect of the invention, the controller further integrates a difference between a first calorie consumption rate and the second calorie consumption rate over the warm up time within the exercise period during which the heart rate is not constant, and the controller calculates the first calorie consumption rate using the detected first heart rate.

According to an aspect of the invention, the controller also integrates a difference between the first calorie consumption rate and the regular calorie consumption rate over the exercise period.

According to an aspect of the invention, the warm up time conforms to ten percent of the exercise period and the second point in the exercise period conforms to ninety percent of the exercise period.

According to an aspect of the invention, the regular component is $T * Y_0$, the warm up exercise component is

(T/10)*(Y$_1$(2)-Y$_1$(1))*0.5, the exercise component is (9T/10)* (Y$_1$(2)-Y$_1$(1)), Y$_0$ = 0.01808 * (X(1)-A+20.25)+C, Y$_1$(1)= B$_m$*(X(1)-A)+C+0.3645,

Y$_1$(2)= B$_m$*(X(2)-A)+C+0.3645, X(1) is the detected first heart rate, X(2) is the detected second heart rate, A is the heart rate while the user is at rest, C is a constant relating to a coefficient of basal metabolic rate per minute, T is the exercise period, B$_m$ = 0.0109*(LBM/H$^2$)-0.0023*(%FAT)-0.0007*(age)-0.0211, LBM is a percent of body mass attributed to muscle, H is height, and age is an age of the user.

According to an aspect of the invention, the regular component is T * Y$_0$, the warm up exercise component is (T/10)*(Y$_1$(2)-Y$_1$(1))*0.5, the exercise component is (9T/10)* (Y$_1$(2)-Y$_1$(1)), Y$_0$ = 0.00895 * (X(1)-A+20.25)+C, Y$_1$(1) = B$_f$ * (X(1)-A)+C+0.1812, Y$_1$(2) = B$_f$ * (X(2)-A)+C+0.1812, X(1) is the detected first heart rate, X(2) is the detected second heart rate, A is the heart rate while the user is at rest, C is a constant relating to a coefficient of basal metabolic rate per minute, T is the exercise period, B$_f$ = 0.0140(LBM/H$^2$)-0.0012(%FAT)-0.1254, LBM is a percent of body mass attributed to muscle, H is height, and age is an age of the user.

According to an aspect of the invention, the device may further include at least one processor including the controller which further reproduces audio and/or video data stored in a memory connected to the measuring device.

According to an aspect of the invention, the at least one processor decodes and outputs audio and/or video data encoded in a format selectable between data encoded according to an MPEG standard and a windows media standard.

According to an aspect of the invention, the device may further include a housing and a battery within the housing which provides power to the at least one processor, wherein the controller is disposed in the housing and the pulse input unit includes an indentation in the housing sized to receive a finger tip.

According to an aspect of the invention, the first point is at a beginning of the exercise period, and the second point is an end of the exercise period.

According to an aspect of the invention, the measuring device includes a hand held portable electrical device.

According to an aspect of the invention, at the first point, the controller notifies the user to take a pulse using the pulse input unit to obtain the first heart rate and, at the second point, the controller notifies the user to take another pulse using the pulse input unit to obtain the second heart rate, and for at least a portion of the exercise period other than at the first and second points, the user does not take a pulse using the pulse input unit.

According to an aspect of the invention, there is provided a method of measuring calorie consumption of a user, the method comprising: detecting a first heart rate of the user at a first point in an exercise period and a second heart rate of the user at a second point in the exercise period other than the first point; and calculating calories consumed during the exercise period using the detected first heart rate, the detected second heart rate, a predetermined at rest heart rate of the user, and one or more factors selected from an age of the user, a gender of the user, a weight of the user, a height of the user, and a percent body mass attributed to muscle of the user, characterized in that the calculating of the calories consumed between the first and second points in the exercise period further uses the detected first heart rate.

According to an aspect of the invention, the calculating the calories consumed includes determining an exercise component corresponding to calories consumed during an exercise period, and the exercise component has a discontinuous rate of change for the heart rate between the first and second periods based upon the detected first and second heart rates.

According to an aspect of the invention, the determining the exercise component includes determining a first calorie consumption component during a first time period substantially at a beginning of the exercise period and during which time the user's heart rate is changing from the detected first heart rate to the detected second heart rate, and determining a second calorie consumption component at a second time period after the first time period and during which time the user's heart rate is substantially the detected second heart rate.

According to an aspect of the invention, the first time period is ten percent of the exercise period and the second time period is ninety percent of the exercise period.

According to an aspect of the invention, the calculating of the calories consumed includes calculating a first calorie consumption rate using the detected first heart rate, calculating a second calorie consumption rate using the detected second heart rate, and determining a warm up exercise component by integrating a difference between the first and second calorie consumption rates over a warm up time within the exercise period when the heart rate is not constant.

According to an aspect of the invention, the determining the warm up exercise component includes calculating a rate of change of the calorie consumption rate during the warm up time to be increasing at a substantially constant rate.

According to an aspect of the invention, the rate of change is a difference between the calculated first and second calorie consumption rates divided by the warm up time.

According to an aspect of the invention, the calculating the calories consumed includes calculating a second calorie consumption rate using the detected second heart rate, calculating a regular calorie consumption rate using an at rest heart rate measured when the user is substantially a time for first awakening, and determining an exercise component of the calories consumed by integrating a difference between the calculated second and regular calorie consumption rates over a steady portion of the exercise period during which the heart rate is constant and after a warm up time during which the heart rate is not constant.

According to an aspect of the invention, a rate of change of the calorie consumption rate during the steady portion after the warm up time is substantially zero.

According to an aspect of the invention, the calculating the calories consumed further includes calculating a first calorie consumption rate using the detected first heart rate, and determining a warm up exercise component by integrating a difference between, the calculated first calorie consumption rate and the calculated second calorie consumption rate over the warm up time within the exercise period during which the heart rare is not constant.

According to an aspect of the invention, the calculating the calories consumed further includes calculating a regular component by integrating a difference between the calculated first and regular consumption rates over the exercise period.

According to an aspect of the invention, the warm up time conforms to ten percent of the exercise period and the second point in the exercise period conforms to ninety percent of the exercise period.

According to an aspect of the invention, the calculating the calories consumed further includes determining a gender of the user, if the user is one of a male and a female, the regular component is $T* Y_0$, the warm up exercise component is $(T/10)*(Y_1(2)-Y_1(1))*0.5$, the exercise component is $(9T/10)* (Y_1(2)-Y_1(1))$, $Y_0 = 0.01808 * (X(1)-A+20.25) +C$, $Y_1(1)= B_m*(X(1)-A)+C+0.3645$, $Y_1(2)= B_m*(X(2)-A)+C+0.3645$, $X(1)$ is the detected first heart rate, $X(2)$ is the detected second heart rate. A is the heart rate while the user is at rest. C is a constant relating to a coefficient of basal metabolic rate per minute, T is the exercise period, $B_m = 0.0109^-(LBM/H^2)-0.0023^-(\%FAT)-0.0007^-(age)-0.0211$, LBM is a percent of body mass attributed to muscle. H is height, and age is an age of the user, and if the user is the other one of the male and the female, the regular component is $T* Y_0$, the warm up exercise component is $(T/10)*(Y_1(2)-Y_1(1))*0.5$, the exercise component is $(9T/10)* (Y_1(2)-Y_1(1))$. $Y_0 = 0.00895* (X(1)-A+20.25)+C$, $Y_1(1) = B_f *(X(1)-A)+C+0.1812$, $Y_1(2) = B_f *(X(2)-A)+C+0.1812$, $X(1)$ is the detected first heart rate, $X(2)$ is the detected second heart rate, A is the heart rate while the user is at rest, C is a constant relating to a coefficient of error per minute, T is the exercise period, $B_f = 0.014.0(LBM/H^2)-0.0012(\%FAT)-0.1254$, LBM is a percent of body mass attributed to muscle, H is height, and age is an age of the user.

The invention also provides a computer program comprising a computer program code adapted to cause the measuring device described above to perform the method described above when said program is run on the controller of said measuring device.

The invention thus provides a method of measuring calorie usage that only uses the heart rates at predetermined times, such as before and after exercise, and does not require continuous monitoring of the heart rates over the exercise period and/or for sustained portions of the exercise period.

The method may be implemented as software using a portable digital audio player having a pulse input unit and which is effective to calculate the calories consumed using discrete measurements of the heart rates before and after exercise.

Additional aspects and/or advantages of the invention will be set forth in part in the description which follows and, in part, will be obvious from the description, or may be learned by practice of the invention.

These and/or other aspects and advantages of the invention will become apparent and more readily appreciated from the following description of the embodiments, taken in conjunction with the accompanying drawings of which:

FIG. 1 shows a portable digital audio player having a calorie measuring device according to an embodiment of the invention;

FIG. 2 is a block diagram showing an interaction between a computer and the calorie measuring device according to an embodiment of the invention;

FIG. 3 shows a display reflecting past exercise periods and the calories consumed as measured using a calorie measuring method according to an embodiment of the invention;

FIGs. 4 and 5 are flow charts of a method of measuring calories according to an embodiment of the invention;

FIG. 6 is a graph showing the integration performed in the method of measuring calories according to an aspect of the invention; and

FIG. 7 is a graph showing an integration performed in another method of measuring calories.

Reference will now be made in detail to the embodiments of the present invention, examples of which are illustrated in the accompanying drawings, wherein like reference numerals refer to the like elements throughout. The embodiments are described below in order to explain the present invention by referring to the figures.

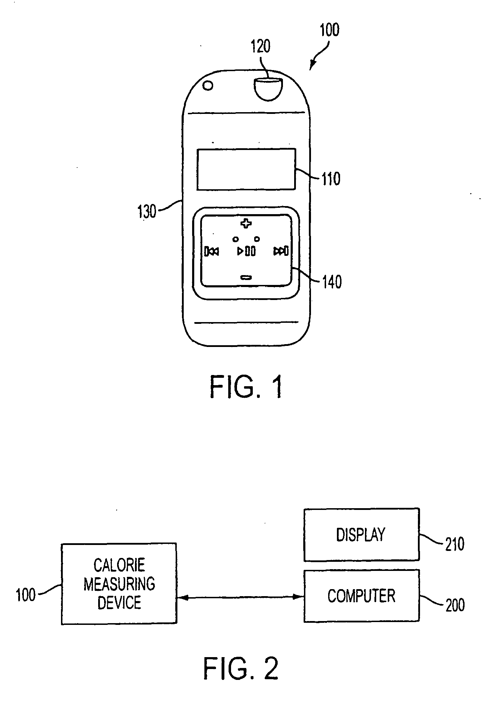

According to an aspect of the invention shown in FIG. 1, a calorie measuring device 100 includes a body 130 having disposed thereon a pulse measuring device 120. As shown, the pulse measuring device 120 measures the pulse based upon a fingertip, which is depressed therein and from which the pulse (i.e., heart rate) is measured. Specifically, the pulse measuring device 120 uses an optical signal adopting photoplethysmography to develop a signal based upon a pulse detected from a finger placed on the pulse measuring device 120.

The shown pulse measuring device 120 is molded to the shape of a finger. However, it is understood that other mechanisms can be used to take a pulse, and that the pulse measuring device 120 need not be integrally connected to the body 130 in all aspects of the invention, and can instead be separately provided so as to connect to and transfer a detected pulse to the calorie measuring device 100.

The calorie measuring device 100 further includes a display 110, on which the measured number of calories are displayed. While not required in all aspects, the display 130 may further display other information processed by the calorie measuring device 100. Further shown are controls 140, which are used to indicate start and stop signals as well as other signals relevant to the operation of the calorie measuring device 100. However, it is understood that the display 110 need not be used in all aspects of the invention, and that the controls 140 need not be integral to the body 130 and instead may be separately provided so as to connect and transfer signals to the calorie measuring device 100. Further, it is understood that the controls 140 can be included in the display 110 where the display 110 includes a touch screen functionality.

As shown, the calorie measuring device 100 may further include a digital audio player, which allows the user to further listen to audio recorded in a digital audio format, such as audio data encoded in MP3, WMV, WMA, secure WMA, and other data formats. For example, the calorie measuring device 100 could be implemented in a modified SPORTS YEPP YP-60, produced by SAMSUNG ELECTRONICS CO., LTD. However, it is understood that other encoding formats can be used, that other types of digital audio players can be used, and that other types of portable digital data recorders can be used. By way of example, it is possible for the calorie device 100 to be included in a recorder having a display 110 suitable for reproducing video, as would be useful for portable DVD players or portable digital video recorders (DVRs) for use in displaying compressed video data. Further, it is understood that the calorie measuring device 100 could be implemented in a personal digital assistant (PDA), or a smart telephone, which includes functionalities from PDA's, and MP3 players and telephones in one unit. Further, it is understood that the calorie measuring device 100 could be implemented as a stand alone device used only for measuring the pulse and/or calories burned by an individual or a watch. It is understood that the present invention can be used in non-portable devices, such as exercise equipment, and need not be used in a portable device.

According to an aspect of the invention shown in FIG. 2, the calorie measuring device 100 interfaces with a general or special purpose computer 200. The interface can be through a wired connection, such as through USB or IEEE 1396 connections and/or through a docking system which is connected to the computer 200, or through wireless systems such as Bluetooth, 802.11 wireless connections or other wireless standards. The computer 200 detects the measured heart rate stored in the calorie measuring device 100 and provides the results on a display 210.

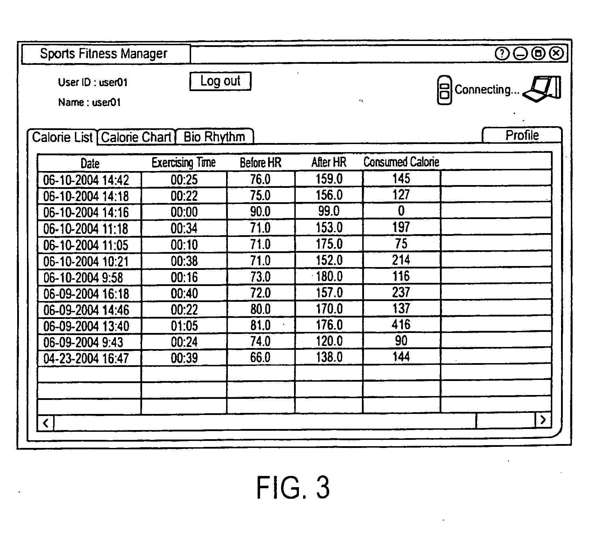

An example of the displayed results is shown in FIG. 3, which shows a Sports Fitness Manager tracking dates, times of exercise, heart rates before and after exercise, and the consumed calories for each particular exercise session. The Sports Fitness Manager also charts the calories being consumed as a function of date, and will also provides a bio rhythm based upon the input birth date. The Sports Fitness Manager is implemented using software included on the computer 200 and imports the calorie consumption information. However, it is understood that the Sports Fitness Manager need not include each of the features shown in FIG. 3, and further need not be included in all aspects of the invention.

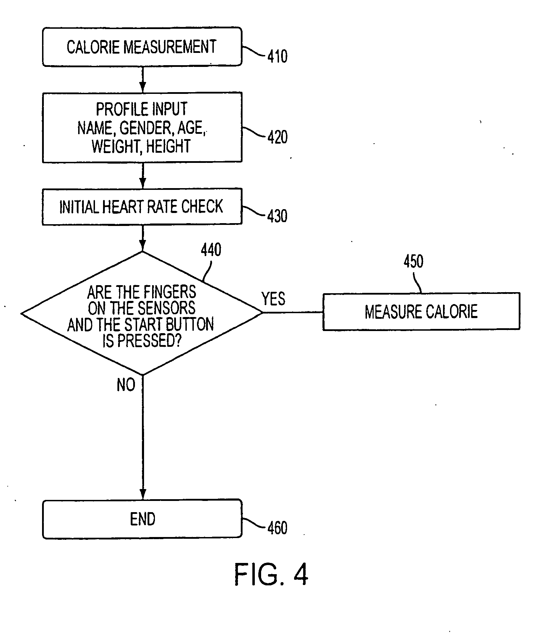

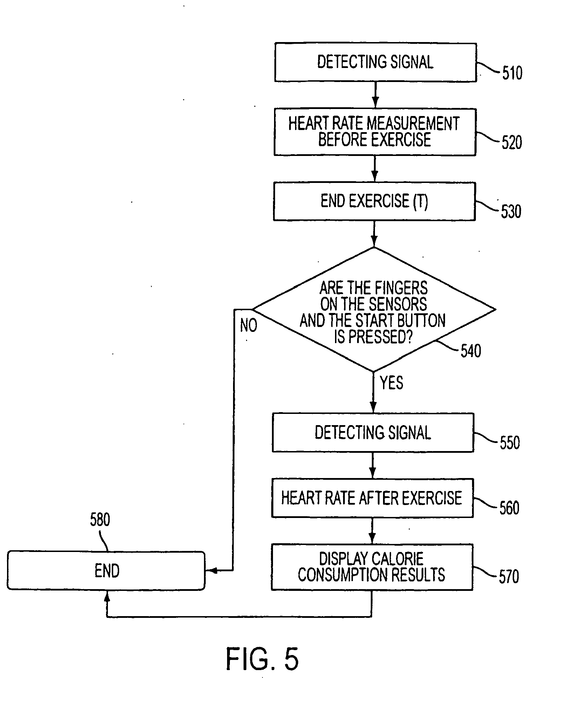

According to an aspect of the invention, the calorie measuring device 100 performs a method as shown in FIGs. 4 and 5. In FIG. 4, the user begins the calorie measuring process by indicating to the calorie measuring device 100 that the program is to begin (operation 410). In operation 420, the user inputs the name, the gender, the age, the weight, and the height of the user who desires to have their calorie measured. Generally, the gender, age, height, and weight are used to calculate a body fat percentage. However, it is understood that the body fat percentage can also be input separately.

The gender, age, height, and weight are input in integer form. However, it is understood that the number can have other forms, and can be input with decimal places or using fractions. Further, this information can be input using the controls 140, or can be input by synchronizing with the Sports Fitness Manager or like program on the computer 200. It is further understood that not all elements need to be input, such as the name, in all aspects of the invention. However it is understood that use of a user name is helpful in order to distinguish cases where multiple people are using the same calorie measuring device 100.

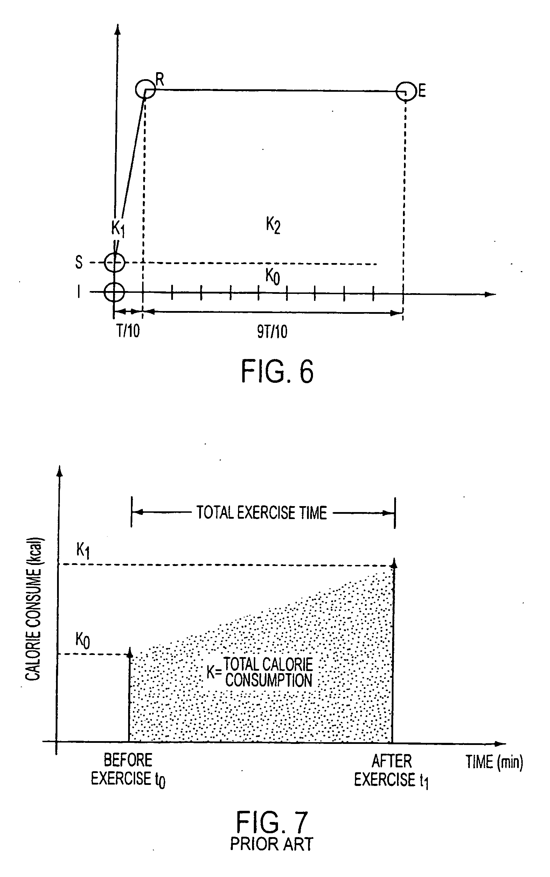

In operation 430, the users initial heart rate is checked. Specifically, the user puts a finger on the pulse measuring device 120, in order to detect an at rest/regular heart rate. The regular heart rates determined in operation 430, as well as the factors entered in operation 420, are used to calculate calories burned during exercise. The regular heart rate of the individual is measured when the individual wakes up and before the individual gets out of bed. The regular heart rate is the regular heart rate is depicted graphically as element I in FIG. 6.

While not required in all aspects of the invention, the regular heart rate is measured in advance of the exercise period and is stored in the calorie measuring device 100. Further, while not required in all aspects of the invention, the input gender, age, weight, and height, and the regular heart rate is stored in a memory of the calorie measuring device 100 for later use. In this way, the individual need not repeat the detection of the regular heart rate in operation 430 and/or enter the gender, age, weight, and height each time the individual uses the calorie measuring device 100. It is understood that, instead of storing this data in an internal memory, one or more of the regular heart rate, gender, age, weight, height and/or name factors can be stored on and retrieved from an external memory, such as a memory stick, which is inserted into the calorie measuring device 100.

When the user wishes to measure calories consumed during an exercise period, the user needs to place a finger on the pulse measuring device 120 and presses a start button on the controls 140 in order to detect a pulse rate at the beginning of an exercise period. As such, in operation 440, the calorie measuring device 100 determines whether the fingers are on the pulse measuring device 120, and whether a start button of the controls 140 is pressed. The operation 440 detects whether the user is about to enter an exercise period for which the calories are to be measured.

If the start button has been pressed and the fingers are on the pulse measuring device 120 (i.e., also referred to generically as sensors), the calorie measuring device 100 measures the calories in operation 450. If the fingers are not on the sensors and the start button has not been pressed, the calorie measurement routine ends. While shown as requiring that the fingers be on the sensors and a start button be pressed in operation 440, it is understood that in aspects of the invention, the calorie measuring device 100 does not need a start button to be pressed, and instead can merely rely on whether the fingers have been detected on the sensors.

Operation 450 will be explained in further detail with regard to FIG. 5 according to an aspect of the invention. In FIG. 5, the calorie measuring device 100 begins detecting the pulse signal from the pulse measuring device 120 so as to detect the pulse before the exercise is begun.

By way of comparison, the heart rate measured before exercise 520 is measured as shown in FIG. 6 as element S, whereas the regular heart rate measured in operation 430 is measured as shown in FIG. 6 as element I.

In operation 530, the user begins exercising and starts the exercise period T. After the exercise period T, the individual again measures the heart rate in operation 540 by putting a finger on the pulse measuring device 120 and pressing the start button. It is understood that the single start button need not be used to implement aspects of the invention, multiple buttons can be used in order to prevent confusion, or a switch can be used to toggle between positions.

If the fingers are on the pulse measuring device 120, and the start button has been pressed in operation 540, the heart rate is detected in operation 550. The heart rate after exercise is measured based on the detected signal in operation 560. As shown in FIG. 6, the heart rate after exercise is shown as element E.

After operation 560, the calorie measuring device 100 calculates the calories consumed as explained below in greater detail, and the results are displayed on the display 110 in operation 570. After the display in 570, the calorie measuring operation is concluded in operation 580. While not required in all aspects, it is understood that the heart rate and the calorie consumed can be stored and/or transferred to the computer 200 in order to create a log of heart rates, and measured calories consumed as shown in FIG. 3. Further, it is understood that operation 570 need not display the calorie results on the display 110 of the calorie measuring device 100, and instead can rely upon the display 210 of the computer 200. Such an embodiment of the invention might be useful where no display 130 is included on the calorie measuring device 100.

If the fingers are not on the sensors and/or the start button is not pressed in operation 540, the operation ends in operation 580. In this way, if the individual loses interest in measuring the calories consumed, the routine is ended. The calorie measuring device 100 can determine whether the calorie measuring routine is be ended if the start button is not pressed within a predetermined time after the exercise is believed to have begun, and if the individual indicates through the controls 140 that the routine is to be terminated. Additionally, if the expected exercise period T is entered by the user at the beginning of the exercise period, if the exercise start button is not pressed within a predetermined period after the expected exercise period T is concluded, the calorie measuring device 100 can conclude the calorie measuring routine in operation 580. However, it is understood that other mechanisms for terminating the routine can be implemented, and that the termination of the routine need not be performed at operation 540 in all aspects of the invention.

According to an aspect of the invention, the calorie measuring device 100 indirectly measures the calorie consumption in operation 570 using the heart rate monitored before and after exercise without requiring measurement of the heart rate during the exercise. As such, while it is possible to utilize additional heart rates measured during exercise, such additional data points are not required.

Specifically, an embodiment of the calorie measuring device 100 uses an indirect calorie measurement technique in which the amount of oxygen consumption is measured based upon the heart rate. Since the amount of oxygen consumed relates to the energy consumed in a time period, it possible to know the amount of calories consumed according to the oxygen consumed. According to an aspect of the invention, the calorie measuring device 100 uses a relationship between the oxygen consumed, user's gender, age, height, weight, and the user's heart rate before and after exercise.

According to an aspect of the invention, the formulas for the calorie consumption rate are provided in Equations 1 and 2. Equation 1 is for calorie consumption rate during rest for both male and females.

### Equation 1

$$\text{Male: } Y_0(\text{kcal/min}) = 0.01808(X-A+20.25)+C$$

$$\text{Female: } Y_0(\text{kcal/min}) = 0.00895(X-A+20.25)+C$$

In Equation 1, the 0.01808 coefficient for Males, and the 0.00895 coefficient for females represents an average of X. These averages are obtained from formulas for calorie consumption during the regular heart rate. Further, A is the regular heart rate measured during operation 430, and X is the heart rate during measurement in operation 520 (i.e., at the beginning of the exercise period T). C is a coefficient of basal metabolic rate per minute. The measurement is in kcal/min, $C = c * a/60$, where c is the Coefficient of regular heart rate per skin area ($kcal/m^2/h$) and a is a surface area of skin ($cm^2$) = W0.444 * H0.663 * 88.83 (for people of 6 years or older). W is the weight (e.g., in kg) and H is the height (e.g., in cm). Calorie consumption is calculated based from the subject's height and the value of a. Further, the value of 20.25 within the Equation 1 formula is the average difference between the regular heart rate in a lying down position and the regular heart rate in an upright position.

Graphically, Equation 1 is shown as area K0 is FIG. 6. Thus, at the beginning of the exercise period T using the heart rate taken in operations 430 and 520, the area K0 is Y1 * T.

Generally, c is a basal metabolic rate in the body as set forth below in Table 1 and further discussed in Japanese patent publication no. 08-52119, based upon Public Welfare Annuity Hospital Annual Report, volume 17, p201-206 (1990), and/or with Company Height Medical Science, Hirosaki Medical Department Report, volume 40-1, p60-69 (1988).

Table 1
Basal Metabolic Rate (kcal/rrf/h)

| Age | Men | Women |
|---|---|---|
| 0 | 48.7 | 48.4 |
| 1 | 53.6 | 52.6 |
| 2 | 56.2 | 56.1 |
| 3 | 57.2 | 55.6 |
| 4 | 56.3 | 54.0 |
| 5 | 55.1 | 51.6 |
| 6 | 52.9 | 49.5 |
| 7 | 51.1 | 47.6 |
| 8 | 49.3 | 46.2 |
| 9 | 47.5 | 44.8 |
| 10 | 46.2 | 44.1 |
| 11 | 45.3 | 43.1 |
| 12 | 44.5 | 42.2 |
| 13 | 43.5 | 41.2 |
| 14 | 42.8 | 39.8 |
| 15 | 41.7 | 38.1 |
| 16 | 41.0 | 36.9 |
| 17 | 40.3 | 36.0 |
| 18 | 39.6 | 35.6 |
| 19 | 38.8 | 35.1 |
| 20-29 | 37.5 | 34.3 |
| 30-39 | 36.5 | 33.2 |
| 40-49 | 35.6 | 32.5 |

(continued)

Basal Metabolic Rate (kcal/rrf/h)

| Age | Men | Women |
|---|---|---|
| 50-59 | 34.8 | 32.0 |
| 60-64 | 34.0 | 31.8 |
| 65-69 | 33.3 | 31.4 |
| 70-74 | 32.6 | 31.1 |
| 75-79 | 31.9 | 30.9 |
| 80- | 30.7 | 30.0 |

Equation 2 is the Formula for calorie consumption rate during exercise for males and females.

## Equation 2

$$\text{Male: } Y_1 = B_m(X-A)+C+0.3645$$

$$\text{Female: } Y_1 = B_f(X-A)+C+0.1812$$

In Equation 2, $B_m$ = 0.0109(LBM/H$^2$)-0.0023(%FAT)-0.0007(age)-0.0211. $B_f$ = 0.0140(LBM/H$^2$)-0.0012(%FAT)-0.1254. X is the heart rate during the exercise period T (i.e., measured in operations 560 and 520), and A is the at rest heart rate before the exercise period T (i.e., measured in operation 430).

LBM is the percent of body mass attributed to muscle and is therefore equal to W(1-%FAT). %FAT is the percent body fat of the individual.

In Equation 2, when looking at the calorie consumption rate during exercise, it is observed that, as compared to the rate during rest, the differences between individual people are apparent. Specifically, it was found that the coefficient of X for men, Bm, is proportional to the weight minus the weight of fat in the body (weight - %FAT*weight) divided by the square of the height. Thus, the coefficient of X for men Bm was inversely proportional with age and percent body fat.

For women, the coefficient of X for men Bf was found to be proportional to (LBM/Ht$^2$) and inversely proportional to percent body fat. However, there was no clear relation with age such that Equation 2 does not include an age factor for women.

While it is possible to directly input body fat values to input the %FAT factor used in equations 1 and 2, the calorie measuring device 100 calculates the %FAT according to the height H, weight W, age A, and gender as follows in Equations 3 through 8. Specifically, Equation 3 is for males of Ages 10~16, Equation 4 is for females of Ages 10~16, Equation 5 is for males of Ages 17~18, Equation 6 is for females of Ages 17~18, Equation 7 is for males of Ages 19 and beyond, and Equation 8 is for females of Ages 19 and beyond.

## Equation 3

$$f = 0.0005870H^2 - 2.761 \times 10^6 \frac{1}{(R-23.091)^2} + 0.0002813 \times 10^6 \frac{H^2}{(R-23.091)^2} + 0.0094R + 0.443W - 12.75$$

## Equation 4

$$f = 0.0004907H^2 - 2.27 \times 10^6 \frac{1}{(R-23.091)^2} + 0.0002275 \times 10^6 \frac{H^2}{(R-23.091)^2} + 0.349W + 2.830$$

## Equation 5

$$f = 0.0005802H^2 - 2.682 \times 10^6 \frac{1}{(R-23.091)^2} + 0.000277 \times 10^6 \frac{H^2}{(R-23.091)^2} + 0.006975R + 0.371W - 6.05806$$

### Equation 6

$$f = 0.0004867H^2 - 2.261 \times 10^6 \frac{1}{(R - 23.091)^2} + 0.0002266 \times 10^6 \frac{H^2}{(R - 23.091)^2} - 0.0026R + 0.299W + 8.1$$

### Equation 7

$$f = 0.0005742H^2 - 2.666 \times 10^6 \frac{1}{(R - 23.091)^2} + 0.0002688 \times 10^6 \frac{H^2}{(R - 23.091)^2} + 0.00369R + 0.3W - 0.09A + 3.149794$$

### Equation 8

$$f = 0.0004871H^2 - 2.286 \times 10^6 \frac{1}{(R - 23.091)^2} + 0.0002250 \times 10^6 \frac{H^2}{(R - 23.091)^2} - 0.0054R + 0.25W - 0.068A + 14.7057$$

In Equations 3 through 8, f = weight - minus weight of fat (i.e., LBM in Equation 2). %FAT = ((weight f)/weight) X 100, and R is the electrical resistance of body. In a male, R is 635, and in a female, R is 833. H is height(cm), W is weight(kg), and A is age.

Using Equations 1 and 8, the calories consumption is obtained directly from the heart rate according to an aspect of the invention. However, it is understood that, if the percent body fat is independently derived and input, only Equations 1 and 2 are used according to an aspect of the invention.

As shown graphically in FIG. 6, the oxygen consumed prior to exercise is related to an area K0, which extends from the regular heart rate at element I and the heart rate at the beginning of exercise at element S and extends for the entire exercise period T. The area K0 is the calorie consumption during rest and relates to the calories consumed in the individual's normal routine. The heart rate at element I corresponds to the regular heart rate, which is generally input ahead of time in operation 430 and is measured up to a minute from when the subject wakes up from sleep in bed, right before the subject gets up. In contrast, the heart rate at element S is measured in operation 520 at the beginning of the exercise period T.

The oxygen consumed during exercise is related to areas K1 and K2, which extend from the heart rate at element S at the beginning of the exercise, to the heart rate at element E at the end of the exercise period T. However, since the heart rate is generally only constant after a warm up period at approximately 1/10 of the exercise period T(i.e., at element R), the oxygen consumed during the warm up period is related to the area K1.

As shown graphically in FIG. 6, the area K1 is depicted as a triangular region extending from the heart rate at element S to the heart rate at element R over a period T/10. The area K1 is the calorie consumption rate after starting exercise up to peak heart rate (10% of total exercise period T). While it might be possible to have the user enter a heart rate substantially at element R at the period T/10 according to an aspect of the invention, the calorie measuring device 100 assumes that the heart rate at element R is the same as the heart rate at the end of the exercise period T at element E. As such, this additional data point is not required.

The area K2 is depicted as a rectangular region extending from the heart rate at element R to the heart rate at element E over a period 9T/10. The area K2 is the calorie consumption rate during a maximum sustained heart rate (90% of the exercise period T). While not required, it is understood that, since the heart rate is constant during this period, instead of measuring the heart rate at element E, the second heart rate could be measured between elements R and element E so long as the end of the exercise period T is measured or input. However, in order to reduce the number of operations a user must perform, the second heart rate according to an aspect of the invention is measured at element E so as to simultaneously record the end of the exercise period T.

In order to determine the calories consumed, the relationship between time and the heart rate is integrated. Using the simplified model shown in FIG. 6, the calorie consumption is measured as the sum of the areas K0 + K1 + K2. Thus, using Equation 1, the heart rate at the beginning of exercise X(S) is measured at element S and the heart rate at rest (A) is measured at element I so as to obtain the calorie consumption rate prior to exercise: Y0. K0 = Y0 * T.

Using Equation 2, the calorie consumption rate at element S (Y1 (S)) is calculated using the heart rate detected in operation 520 at the beginning of the exercise period T in order to obtain element S, and the calorie consumption rate at element E (Y1 (E)) is detected in operation 560 at the end of the exercise period T. Thus, K1 = ((Y1(E)-Y(Y1(S)) *0.1*T)*0.5, and K2 = ((Y1 (E)-Y(Y1(8))*0.9*T.

In FIG. 6, element I is at t = 0, hr_0, element S is at t=0, hr_1, element R is at T/10, hr_2, and element E is at t= T, hr_2. Further, hr_0 is the heart rate at rest, hr_1 is the heart rate before exercise, and hr_2 is the heart rate after exercise. T is the total exercise period. From the above, the Total Calorie Consumption can be calculated as K0 + K1 + K2.

Experimentally, the number of calories consumed calculated by the calorie measuring device 100, implemented using a modified sports YEPP YP-60 made by SAMSUNG ELECTRONICS CO., LTD, modified according to an aspect of the invention, were compared against the number of calories consumed as measured using three reference devices: the POLAR A3, made by POLAR of Finland, a treadmill, and the unmodified YEPP YP-60 released February 2004 using an old method shown graphically in FIG. 7 (referred to in Tables 2 and 3 as the "Old Method"). The subjects in these tests were 10 adult males and females in their 20s and 30s in a physical training area.

In regards to the Old Method used to calculate calories consumed, the calories consumed are measured based upon Equation 9:

Equation 9:

$$K(\text{Total Calorie Consumption}) = t_1 k_0 + 05 t_1 (k_1 - k_0).$$

In Equation 9, $t_1$ is the time at the conclusion of the exercise period, $k_1$ is the calories consumed at the end of the exercise period, $t_0$ is the time at the beginning of the exercise period, and $k_0$ is the calories consumed at the beginning of the exercise period. As can be seen in Equation 9 and graphically in FIG. 7, the Old Method assumes a constant slope of the consumed calories as a function of time from a beginning of the exercise period to an end of the exercise period and therefore does not account for a change in the slope occurring at element R shown in FIG. 6. In contrast, the calorie measuring device 100 according to an aspect of the invention accounts for the change in slope occurring after a warm up period.

In addition, the method performed by the POLAR A3 device involves continuous heart monitoring during exercise. As such, while the calorie measuring device 100 shown in FIG. 1 uses an optical signal adopting photoplethysmography to develop a signal based upon a pulse detected from a finger placed on the pulse measuring device 120, the POLAR A3 device uses an electrocardiogram to generate an electrical signal based upon a sensor placed on the chest of the user. While, the POLAR A3 device conducts continuous heart rate monitoring during exercise, the POLAR 43 device is more invasive in requiring a transmitter to be attached to the user's chest during the exercise period and is also expensive. In contrast, the calorie measuring device 100 is able to simply measure the calories consumed in a non-intrusive way by measuring heart rates before and after exercise while also maintaining a low price.

Further, the conventional treadmill measures the number of calories consumed based upon the speed of the treadmill and the time on the treadmill as opposed to using the heart rate.

The results of the experiment are provided in Tables 2 and 3.

Table 2 Comparison of Total Calorie Consumption During Exercise

|  |  | Exercise Type | Old method (Kcal) | New method (Kcal) | Polar (Kcal) | Treadmill (Kcal) |
|---|---|---|---|---|---|---|
|  | subject 01 | Running | 88 | 115 | 132 | 94 |
|  | subject 02 | Running | 151 | 363 | 300 | 204 |
|  | subject 03 | Running | 268 | 356 | 413 | 396 |
|  | subject 04 | Running | 159 | 291 | 292 | 239 |
|  | subject 05 | Running | 142 | 245 | 254 | 261 |
|  | subject 06 | Power Walking | 187 | 268 | 342 | 237 |
|  | subject 07 | Power Walking | 111 | 151 | 163 | 158 |
|  | subject 08 | Power Walking | 152 | 180 | 221 | 208 |
|  | subject 09 | Running+Power Walking | 173 | 270 | 289 | 287 |
|  | subject 10 | Running+Power Walking | 146 | 262 | 279 | 242 |

Table 3: Residual analysis of Total Calorie Consumption During Exercise (Residual = Reference device - Test device)

| | Exercise Type | Polar vs. Old Method (kcal) | Polar vs. New Method (kcal) | Treadmill vs. Old Method (kcal) | Treadmill vs. New Method (kcal) | Treadmill vs. Polar (kcal) |
|---|---|---|---|---|---|---|
| subject 01 | Running | 44 | 17 | 6 | -21 | -38 |
| subject 02 | Running | 149 | -63 | 53 | -159 | -96 |
| subject 03 | Running | 145 | 57 | 128 | 40 | -17 |
| subject 04 | Running | 133 | 1 | 80 | -52 | -53 |
| subject 05 | Running | 112 | 9 | 119 | 16 | 7 |
| subject 07 | Power Walking | 155 | 74 | 50 | -31 | -105 |
| subject 07 | Power Walking | 52 | 12 | 47 | 7 | -5 |
| subject 08 | Power Walking | 69 | 41 | 56 | 28 | -13 |
| subject 09 | Running+ Power Walking | 116 | 19 | 114 | 17 | -2 |
| subject 10 | Running+ Power Walking | 133 | 17 | 96 | -20 | -37 |
| Average | | 110.8 | 18.4 | 74.9 | -17.5 | -35.9 |
| Standard Deviation | | 41.2 | 36.7 | 39.1 | 57.4 | 38.7 |

Based upon the above results, the relative accuracy of the total calorie consumption for the calorie measuring device 100 implemented in the modified YEPP YP-60 according to an aspect of the invention (i.e., the test device) were made while exercise was being performed. The results are provided in Table 4. In Table 4, the Measurement Percent Error (%) = (|Cr - Ct| / Cr) * 100, where Cr is the calorie consumption rate from a reference measurement device, and Ct is the calorie consumption rate from the calorie measuring device 100 (i.e., the testing device).

Table 4: Comparison of Accuracy Total Calorie Consumption During Exercise

| Reference Device | Testing Device Percent Error (%) |
|---|---|
| POLAR A3 | 11.18 |
| Treadmill | 18.34 |

By way comparison, the treadmill Percent Error with reference to the POLAR A3 device was 18.63%, and the POLAR A3 percent Error with reference to the Treadmill was 13.94%. As a result of comparing between the calorie measurements from the POLAR device and measurements from the treadmill, it was observed that the error percentage with respect to the calorie measuring device 100 on both the POLAR A3 and the treadmill is less than the error percentage between the POLAR A3 and the treadmill. As such, the calorie measuring device 100 of the present invention provides comparable results to the POLAR A3 and the treadmill at a greatly reduced cost using a portable device having a non-invasive sensor.

While not required, it is understood that the method of the present invention can be implemented using computer software/processing instructions stored on a computer readable medium. Such software can be distributed on a computer readable medium, implemented as firmware, and/or downloaded to upgrade an existing apparatus to include the calorie measuring method of the present invention.

According to an aspect of the invention, it is possible to simply measure the calorie consumption by measuring the heart rate before and after exercising. According to an aspect of the invention, by using the heart rate measured before exercising, it can instruct the user the most appropriate exercise intensity and time by measuring the calorie consumption during exercise. According to an aspect of the invention, it is possible to apply the calorie consumption measuring device on other portable platforms, as well as other non-portable platforms.

Although a few embodiments of the present invention have been shown and described, it would be appreciated by those skilled in the art that changes may be made to these embodiments without departing from the invention, the scope of which is defined in the claims.

**Claims**

1. A measuring device for use in measuring calories consumed by a user, comprising:

   a pulse input unit (120) arranged to detect a first heart rate of the user at a first point in an exercise period and a second heart rate of the user at a second point in the exercise period other than the first point, the pulse input unit (120) not detecting a heart rate between the first and second points; and

   a controller arranged to receive the detected first and second heart rates and calculates calories consumed between the first and second points in the exercise period using the detected second heart rate, a predetermined at rest heart rate of the user, and one or more factors selected from an age of the user, a gender of the user, a weight of the user, a height of the user, and a percent of the body weight attributed to muscle of the user, **characterized in that** the controller is arranged to calculate the calories consumed between the first and second points in the exercise period further using the detected first heart rate.

2. The measuring device of claim 1, wherein the controller is arranged to calculate different rates of change of the heart rate during the exercise period based upon the detected first and second heart rates.

3. The measuring device of claim 2, wherein the controller is arranged to calculate:

   a first calorie consumption component having a first rate of change during a first time period at a beginning of the exercise period and during which time the user's heart rate is changing from the detected first heart rate to the detected second heart rate, and

   a second calorie consumption component having a second rate of change at a second time period after the first time period and during which time the user's heart rate is substantially the detected second heart rate.

4. The measuring device of claim 3, wherein the first time period is ten percent of the exercise period and the second time period is ninety percent of the exercise period.

5. The measuring device of claim 1, wherein:

   the controller is arranged to calculate a first calorie consumption rate using the detected first heart rate, and a second calorie consumption rate using the detected second heart rate, and

   the controller is arranged to integrate a difference between the calculated first and second calorie consumption rates over a warm up time during which the heart rate is not constant in order to calculate calories consumed during the warm up time of the exercise period.

6. The measuring device of claim 5, wherein the controller is arranged to calculate a rate of change of the calorie consumption rate during the warm up time to be increasing at a constant rate.

7. The measuring device of claim 6, wherein the controller is arranged to calculate the rate of change to be a difference between the calculated first and second calorie consumption rates divided by the warm up time.

8. The measuring device of claim 1, wherein:

   the controller is arranged to calculate a second calorie consumption rate using the detected second heart rate, the controller is arranged to calculate a regular calorie consumption rate using the predetermined at rest heart rate, the predetermined at rest heart rate being a heart rate substantially at a time when the user first awakes from sleep, and

   the controller is arranged to integrate a difference between the calculated second and regular calorie consumption rates over a steady portion of the exercise period during which the heart rate is constant and after a warm up time during which the heart rate is not constant.

9. The measuring device of claim 8, wherein a rate of change of the calorie consumption rate during the steady portion is zero.

10. The measuring device of claim 9, wherein the controller is arranged to further integrate a difference between a first calorie consumption rate and the second calorie consumption rate over the warm up time within the exercise period during which the heart rate is not constant, and
the controller is arranged to calculate the first calorie consumption rate using the detected first heart rate.

11. The measuring device of claim 10, wherein:

the controller is also arranged to integrate a difference between the first calorie consumption rate and the regular calorie consumption rate over the exercise period.

12. The measuring device of claim 11, wherein the warm up time conforms to ten percent of the exercise period and the second point in the exercise period conforms to ninety percent of the exercise period.

13. The measuring device of claim 12, wherein:

the regular component is $T * Y_0$,
the warm up exercise component is $(T/10)*(Y_1(2)-Y_1(1))*0.5$,
the exercise component is $(9T/10)* (Y_1(2)-Y_1(1))$,

where:

$$Y_0 = 0.01808 * (X(1)-A+20.25)+C,$$

$$Y_1(1)= B_m*(X(1)-A)+C+0.3645,$$

$$Y_1(2)= B_m*(X(2)-A)+C+0.3645,$$

X(1) is the detected first heart rate,
X(2) is the detected second heart rate,
A is the heart rate while the user is at rest,
C is a constant relating to a coefficient of basal metabolic rate per minute,
T is the exercise period,

$$B_m = 0.0109*(LBM/H^2)-0.0023*(\%FAT)-0.0007*(age)-0.0211,$$

LBM is the percent of body mass attributed to muscle,
H is the height, and
age is the age of the user.

14. The measuring device of claim 11, wherein:

the regular component is $T * Y_0$,
the warm up exercise component is $(T/10)*(Y_1(2)-Y_1(1))*0.5$,
the exercise component is $(9T/10)* (Y_1(2)-Y_1(1))$,

where:

$$Y_0 = 0.00895 * (X(1)-A+20.25)+C,$$

$$Y_1(1) = B_f * (X(1)-A)+C+0.1812,$$

$$Y_1(2) = B_f * (X(2)-A)+C+0.1812,$$

X(1) is the detected first heart rate,
X(2) is the detected second heart rate,
A is the heart rate while the user is at rest,
C is a constant relating to a coefficient of basal metabolic rate per minute,
T is the exercise period,

$$B_f = 0.0140(LBM/H^2)-0.0012(\%FAT)-0.1254,$$

LBM is the percent of body mass attributed to muscle,
H is the height, and
age is the age of the user.

15. The measuring device of any preceding claim, wherein the controller is at least one processor, which processor is arranged to reproduce audio and/or video data stored in a memory connected to the measuring device.

16. The measuring device of claim 15, further comprising a housing (130) and a battery within the housing (130) arranged to provide power to the at least one processor, wherein the controller is disposed in the housing (130) and the pulse input unit comprises an indentation in the housing (130) sized to receive a finger tip.

17. The measuring device of any preceding claim, wherein the first point is at a beginning of the exercise period, and the second point is an end of the exercise period.

18. A hand held portable electrical device (100) comprising the measuring device of any preceding claim.

19. The measuring device of any of claims 1 to 17, wherein:

at the first point, the controller notifies the user to take a pulse using the pulse input unit (120) to obtain the first heart rate and, at the second point, the controller notifies the user to take another pulse using the pulse input unit (120) to obtain the second heart rate, and
for at least a potion of the exercise period other than at the first and second points, the user does not take a pulse using the pulse input unit (120).

20. The measuring device of any of claims 1 to 17, wherein the pulse input unit (120) is arranged to detect the first heart rate and the second heart rate using a photoplethsmography signal measured from a finger of the user.

21. A method of measuring calorie consumption of a user, the method comprising:

detecting a first heart rate of the user (520) at a first point in an exercise period and a second heart rate of the user (560) at a second point in the exercise period other than the first point; and
calculating calories consumed during the exercise period using the detected first heart rate, the detected second heart rate, a predetermined at rest heart rate of the user, and one or more factors selected from an age of the user, a gender of the user, a weight of the user, a height of the user, and a percent body mass attributed to muscle of the user,
**characterized in that** the calculating of the calories consumed between the first and second points in the exercise period further uses the detected first heart rate.

22. The method of claim 21, wherein:

the calculating of the calories consumed comprises determining an exercise component corresponding to calories consumed during an exercise period, and

the exercise component has a discontinuous rate of change for the heart rate between the first and second periods based upon the detected first and second heart rates.

23. The method of claim 22, wherein the determining of the exercise component comprises:

determining a first calorie consumption component during a first time period at a beginning of the exercise period and during which time the user's heart rate is changing from the detected first heart rate to the detected second heart rate, and
determining a second calorie consumption component at a second time period after the first time period and during which time the user's heart rate is substantially the detected second heart rate.

24. The method of claim 23, wherein the first time period conforms to ten percent of the exercise period and the second time period conforms to ninety percent of the exercise period.

25. The method of claim 21, wherein the calculating of the calories consumed comprises:

calculating a first caolorie consumption rate using the detected first heart rate,
calculating a second calorie consumption rate using the detected second heart rate, and
determining a warm up exercise component by integrating a difference between the first and second calorie consumption rates over a warm up time within the exercise period when the heart rate is not constant.

26. The method of claim 25, wherein the determining of the warm up exercise component comprises calculating a rate of change of the calorie consumption rate during the warm up time to be increasing at a constant rate.

27. The method of claim 26, wherein the rate of change is a difference between the calculated first and second calorie consumption rates divided by the warm up time.

28. The method of claim 21, wherein the calculating of the calories consumed comprises:

calculating a second calorie consumption rate using the detected second heart rate,
calculating a regular calorie consumption rate using the predetermined at rest heart rate measured when the user is substantially at a time for first awakening, and
determining an exercise component of the calories consumed by integrating a difference between the calculated second and regular calorie consumption rates over a steady portion of the exercise period during which the heart rate is constant and after a warm up time during which the heart rate is not constant.

29. The method of claim 28, wherein a rate of change of the calorie consumption rate of the steady portion after the warm up time is zero.

30. The method of claim 29, wherein the calculating of the calories consumed further comprises:

calculating a first calorie consumption rate using the detected first heart rate, and
determining a warm up exercise component by integrating a difference between the calculated first calorie consumption rate and the calculated second calorie consumption rate over the warm up time within the exercise period during which the heart rate is not constant.

31. The method of claim 30, wherein the calculating of the calories consumed further comprises calculating a regular component by integrating a difference between the calculated first and regular consumption rates over the exercise period.

32. The method of claim 31, wherein the warm up time conforms to ten percent of the exercise period and the second point of the exercise period conforms to ninety percent of the exercise period.

33. The method of claim 32, wherein the calculating of the calories consumed further comprises:

determining a gender of the user;
if the user is a male:

the regular component is $T^* Y_0$,
the warm up exercise component is $(T/10)^*(Y_1(2)-Y_1(1))^*0.5$,
the exercise component is $(9T/10)^* (Y_1(2)-Y_1(1))$,
where:

$$Y_0 = 0.01808 * (X(1)-A+20.25)+C,$$

$$Y_1(1)= B_m^*(X(1)-A)+C+0.3645,$$

$$Y_1(2)= B_m^*(X(2)-A)+C+0.3645,$$

X(1) is the detected first heart rate,
X(2) is the detected second heart rate,
A is the heart rate while the user is at rest,
C is a constant relating to a coefficient of basal metabolic rate per minute,
T is the exercise period,

$$B_m = 0.0109^*(LBM/H^2)-0.0023^*(\%FAT)-0.0007^*(age)-0.0211,$$

LBM is the percent of body mass attributed to muscle,
H is the height, and
age is the age of the user; and

if the user is a female:

the regular component is $T^* Y_0$,
the warm up exercise component is $(T/10)^*(Y_1(2)-Y_1(1))^*0.5$,
the exercise component is $(9T/10)^* (Y_1(2)-Y_1(1))$,
where:

$$Y_0 = 0.00895 * (X(1)-A+20.25)+C,$$

$$Y_1(1) = B_f * (X(1)-A)+C+0.1812,$$

$$Y_1(2) = B_f * (X(2)-A)+C+0.1812,$$

X(1) is the detected first heart rate,
X(2) is the detected second heart rate,
A is the heart rate while the user is at rest,
C is a constant relating to a coefficient of basal metabolic rate per minute,
T is the exercise period,

$$B_f = 0.0140(LBM/H^2)-0.0012(\%FAT)-0.1254,$$

LBM is the percent of body mass attributed to muscle,
H is the height, and
age is the age of the user.

**EP 1 647 227 B1**

34. The method of any of claims 21 to 33, wherein the first heart rate and the second heart rate are detected using a photoplethsmography signal measured from a finger of the user.

35. A computer program comprising a computer program code adapted to cause the measuring device of any of claims 1 to 20 to perform the steps of any of claims 21 to 34 when said program is run on the controller of said measuring device.

36. The computer program of claim 35 embodied on a computer readable medium.

**Patentansprüche**

1. Messgerät für den Gebrauch beim Messen von von einem Benutzer verbrauchten Kalorien, die Folgendes umfasst:

   eine Pulseingabeeinheit (120) zum Erfassen einer ersten Herzfrequenz des Benutzers an einem ersten Punkt in einer Trainingsperiode und einer zweiten Herzfrequenz des Benutzers an einem zweiten Punkt in der Trainingsperiode, der sich von dem ersten Punkt unterscheidet, wobei die Pulseingabeeinheit (120) die Herzfrequenz zwischen dem ersten und dem zweiten Punkt nicht erfasst; und
   eine Steuerung zum Empfangen der erfassten ersten und zweiten Herzfrequenzen und zum Berechnen von Kalorien, die zwischen dem ersten und dem zweiten Punkt in der Trainingsperiode verbraucht wurden, anhand der erfassten zweiten Herzfrequenz, einer vorbestimmten Ruheherzfrequenz des Benutzers und eines oder mehrerer Faktoren, die ausgewählt sind aus dem Alter des Benutzers, dem Geschlecht des Benutzers, dem Gewicht des Benutzers, der Größe des Benutzers und einem Prozentanteil des den Muskeln des Benutzers zugeordneten Körpergewichts,
   **dadurch gekennzeichnet, dass** die Steuerung die Aufgabe hat, die zwischen dem ersten und dem zweiten Punkt der Trainingsperiode verbrauchten Kalorien weiter anhand der erfassten ersten Herzfrequenz zu berechnen.

2. Messgerät nach Anspruch 1, wobei die Steuerung die Aufgabe hat, unterschiedliche Herzfrequenzänderungsraten während der Trainingsperiode auf der Basis der erfassten ersten und zweiten Herzfrequenz zu berechnen.

3. Messgerät nach Anspruch 2, wobei die Steuerung die Aufgabe hat, Folgendes zu berechnen:

   eine erste Kalorienverbrauchskomponente mit einer ersten Änderungsrate während einer ersten Zeitperiode zu Beginn der Trainingsperiode, während der sich die Herzfrequenz des Benutzers von der erfassten ersten Frequenz auf die erfasste zweite Herzfrequenz ändert, und
   eine zweite Kalorienverbrauchskomponente mit einer zweiten Änderungsrate in einer zweiten zeitperiode nach der ersten Zeitperiode, während der die Herzfrequenz des Benutzers im Wesentlichen die erfasste zweite Herzfrequenz ist.

4. Messgerät nach Anspruch 3, wobei die erste Zeitperiode zehn Prozent der Trainingsperiode und die zweite Zeitperiode neunzig Prozent der Trainingsperiode ausmacht.

5. Messgerät nach Anspruch 1, wobei:

   die Steuerung die Aufgabe hat, eine erste Kalorienverbrauchsrate anhand der erfassten ersten Herzfrequenz und eine zweite Kalorienverbrauchsrate anhand der erfassten zweiten Herzfrequenz zu berechnen, und
   die Steuerung die Aufgabe hat, eine Differenz zwischen der berechneten ersten und zweiten Kalorienverbrauchsrate über eine Aufwärmzeit zu integrieren, während der die Herzfrequenz nicht konstant ist, um während der Aufwärmzeit der Trainingsperiode verbrauchte Kalorien zu berechnen.

6. Messgerät nach Anspruch 5, wobei die Steuerung die Aufgabe hat, eine Änderungsrate der Kalorienverbrauchsrate während der Aufwärmzeit zu berechnen, die mit einer konstanten Geschwindigkeit zunehmen soll.

7. Messgerät nach Anspruch 6, wobei die Steuerung die Aufgabe hat, die Änderungsrate als eine Differenz zwischen den berechneten ersten und zweiten Kalorienverbrauchsraten dividiert durch die Aufwärmzeit zu berechnen.

8. Messgerät nach Anspruch 1, wobei:

17

die Steuerung die Aufgabe hat, eine zweite Kalorienverbrauchsrate anhand der erfassten zweiten Herzfrequenz zu berechnen,
die Steuerung die Aufgabe hat, eine reguläre Kalorienverbrauchsrate anhand der vorbestimmten Ruheherzfrequenz zu berechnen, wobei die vorbestimmte Ruheherzfrequenz eine Herzfrequenz im wesentlichen zu einer Zeit ist, wenn der Benutzer zuerst vom Schlaf erwacht, und
die Steuerung die Aufgabe hat, eine Differenz zwischen der berechneten zweiten und regulären Kalorienverbrauchsrate über einen steten Teil der Trainingsperiode zu integrieren, während der die Herzfrequenz konstant ist, und nach einer Aufwärmzeit, während der die Herzfrequenz nicht konstant ist.

9. Messgerät nach Anspruch 8, wobei eine Änderungsrate der Kalorienverbrauchsrate während des steten Teils null ist.

10. Messgerät nach Anspruch 9, wobei die Steuerung die Aufgabe hat, eine Differenz zwischen einer ersten Kalorienverbrauchsrate und der zweiten Kalorienverbrauchsrate über die Aufwärmzeit innerhalb der Trainingsperiode zu integrieren, während der die Herzfrequenz nicht konstant ist, und
die Steuerung die Aufgabe hat, die erste Kalorienverbrauchsrate anhand der erfassten ersten Herzfrequenz zu berechnen.

11. Messgerät nach Anspruch 10, wobei;
die Steuerung auch die Aufgabe hat, eine Differenz zwischen der ersten Kalorienverbrauchsrate und der regulären Kalorienverbrauchsrate über die Trainingsperiode zu integrieren.

12. Messgerät nach Anspruch 11, wobei die Aufwärmzeit zehn Prozent der Trainingsperiode ausmacht und der zweite Punkt in der Trainingsperiode neunzig Prozent der Trainingsperiode entspricht.

13. Messgerät nach Anspruch 12, wobei;

die reguläre Komponente $T*Y_0$ ist,
die Aufwärmtrainingskomponente $(T/10)*(Y_1(2)-Y_1(1))*0,5$ ist,
die Trainingskomponente $(9T/10)*(Y_1(2)-Y_1(1))$ ist,
wobei:

$$Y_0 = 0,01808*(X(1)-A+20,25)+C,$$

$$Y_1(1) = B_m*(X(1)-A)+C+0,3645,$$

$$Y_1(2) = B_m*(X(2)-A)+C+0,3645,$$

X(1) die erfasste erste Herzfrequenz ist,
X(2) die erfasste zweite Herzfrequenz ist,
A die Herzfrequenz im Ruhezustand des Benutzers ist,
C eine Konstante in Bezug auf einen Koeffizienten für den Kaloriengrundumsatz pro Minute ist,
T die Trainingsperiode ist,

$$B_m = 0,0109*(LBM/H^2)-0,0023*(\%FAT)-0,0007*(age)-0,0211,$$

LBM der Prozentanteil der den Muskeln zugeordneten Körpermasse ist,
H die Größe ist und
Age das Alter des Benutzers ist.

14. Messgerät nach Anspruch 11, wobei:

die reguläre Komponente $T*Y_0$ ist,
die Aufwärmtrainingskomponente $(T/10)*(Y_1(2)-Y_1(1))*0,5$ ist,

die Trainingskomponente (9T/10)*(Y$_1$(2)-Y$_1$(1)) ist,
wobei:

$$Y_0 = 0,00895*(X(1)-A+20,25)+C,$$

$$Y_1(1)=B_f*(X(1)-A)+C+0,1812,$$

$$Y_1(2)=B_f*(X(2)-A)+C+0,1812,$$

X(1) die erfasste erste Herzfrequenz ist,
X(2) die erfasste zweite Herzfrequenz ist,
A die Herzfrequenz im Ruhezustand des Benutzers ist,
C eine Konstante in Bezug auf einen Koeffizienten für den Kaloriengrundumsatz pro Minute ist,
T die Trainingsperiode ist,

$$B_f = 0,0140*(LBM/H^2)-0,0012*(\%FAT)-0,1254,$$

LBM der Prozentanteil der den Muskeln zugeordneten Körpermasse ist,
H die Größe ist und
Age das Alter des Benutzers ist.

15. Messgerät nach einem der vorherigen Ansprüche, wobei die Steuerung wenigstens ein Prozessor ist, wobei der Prozessor die Aufgabe hat, in einem mit dem Messgerät verbundenen Speicher gespeicherte Audio- und/oder Videodaten zu reproduzieren.

16. Messgerät nach Anspruch 15, die ferner ein Gehäuse (130) und eine Batterie in dem Gehäuse (130) umfasst, um den wenigstens einen Prozessor mit Strom zu speisen, wobei die Steuerung in dem Gehäuse (130) angeordnet ist und die Pulseingabeeinheit eine Vertiefung in dem Gehäuse (130) umfasst, die so bemessen ist, dass sie eine Fingerspitze aufnimmt.

17. Messgerät nach einem der vorherigen Ansprüche, wobei der erste Punkt zu Beginn der Trainingsperiode und der zweite Punkt am Ende der Trainingsperiode ist.

18. Tragbares elektrisches Handgerät (100), das das Messgerät nach einem der vorherigen Ansprüche umfasst.

19. Messgerät nach einem der Ansprüche 1 bis 17, wobei:

die Steuerung an dem ersten Punkt den Benutzer anweist, seinen Puls mit der Pulseingabeeinheit (120) zu messen, um die erste Herzfrequenz zu erhalten, und die Steuerung den Benutzer an dem zweiten Punkt anweist, den Puls mit der Pulseingabeeinheit (120) nochmals zu messen, um die zweite Herzfrequenz zu erhalten, und der Benutzer den Puls mit der Pulseingabeeinheit (120) für wenigstens einen Teil der Trainingsperiode, der nicht am ersten oder am zweiten Punkt ist, nicht misst.

20. Messgerät nach einem der Ansprüche 1 bis 17, wobei die Pulseingabeeinheit (120) die Aufgabe hat, die erste Herzfrequenz und die zweite Herzfrequenz anhand eines an einem Finger des Benutzers gemessenen Photoplethysografiesignals zu erfassen.

21. Verfahren zum Messen des Kalorienverbrauchs eines Benutzers, wobei das Verfahren die folgenden Schritte beinhaltet:

Erfassen einer ersten Herzfrequenz des Benutzers (520) an einem ersten Punkt in einer Trainingsperiode und einer zweiten Herzfrequenz des Benutzers (560) an einem zweiten Punkt in der Trainingsperiode, der sich von dem ersten Punkt unterscheidet; und

Berechnen von Kalorien, die während der Trainingsperiode verbraucht wurden, anhand der erfassten ersten Herzfrequenz, der erfassten zweiten Herzfrequenz, einer vorbestimmten Ruheherzfrequenz des Benutzers sowie eines oder mehrerer Faktoren, die ausgewählt sind aus dem Alter des Benutzers, dem Geschlecht des Benutzers, dem Gewicht des Benutzers, der Größe des Benutzers und einer den Muskeln des Benutzers zugeordneten prozentualen Körpermasse,

**dadurch gekennzeichnet, dass** beim Berechnen der zwischen dem ersten und dem zweiten Punkt in der Trainingsperiode verbrauchten Kalorien ferner die erfasste erste Herzfrequenz benutzt wird.

22. Verfahren nach Anspruch 21, wobei:

das Berechnen der verbrauchten Kalorien das Ermitteln einer Trainingskomponente umfasst, die Kalorien entspricht, die während einer Trainingsperiode verbraucht wurden, und
die Trainingskomponente eine diskontinuierliche Änderungsrate für die Herzfrequenz zwischen der ersten und der zweiten Periode auf der Basis der ersten und der zweiten Herzfrequenzen hat.

23. Verfahren nach Anspruch 22, wobei das Ermitteln der Trainingskomponente Folgendes beinhaltet:

Ermitteln einer ersten Kalorienverbrauchskomponente während einer ersten Zeitperiode zu Beginn der Trainingsperiode, während der sich die Herzfrequenz des Benutzers von der ersten Herzfrequenz auf die erfasste zweite Herzfrequenz ändert, und
Ermitteln einer zweiten Kalorienverbrauchskomponente zu einer zweiten Zeitperiode nach der ersten Zeitperiode, während der die Herzfrequenz des Benutzers im Wesentlichen die zweite erfasste Herzfrequenz ist.

24. Verfahren nach Anspruch 23, wobei die erste zeitperiode zehn Prozent der Trainingsperiode und die zweite Zeitperiode neunzig Prozent der Trainingsperiode ausmacht.

25. Verfahren nach Anspruch 21, wobei die Berechnung der verbrauchten Kalorien Folgendes beinhaltet:

Berechnen einer ersten Kalorienverbrauchsrate anhand der erfassten ersten Herzfrequenz,
Berechnen einer zweiten Kalorienverbrauchsrate anhand der erfassten zweiten Herzfrequenz, und
Ermitteln einer Aufwärmtrainingskomponente durch Integrieren einer Differenz zwischen der ersten und der zweiten Kalorienverbrauahsrate über eine Aufwärmzeit innerhalb der Trainingsperiode, wenn die Herzfrequenz nicht konstant ist.

26. verfahren nach Anspruch 25, wobei das Ermitteln der Aufwärmtrainingskomponente das Berechnen einer Änderungsrate der Kalorienverbrauchsrate während der Aufwärmzeit beinhaltet, die mit einer konstanten Geschwindigkeit zunehmen soll.

27. Verfahren nach Anspruch 26, wobei die Änderungsrate eine Differenz zwischen der berechneten ersten und zweiten Kalorienverbrauchsrate dividiert durch die Aufwärmzeit ist.

28. Verfahren nach Anspruch 21, wobei die Berechnung der verbrauchten Kalorien Folgendes beinhaltet:

Berechnen einer zweiten Kalorienverbrauchsrate anhand der erfassten zweiten Herzfrequenz,
Berechnen einer regulären Kalorienverbrauchsrate anhand der vorbestimmten Ruheherzfrequenz, die im Wesentlichen beim ersten Erwachen des Benutzers gemessen wird, und
Ermitteln einer Trainingskomponente der verbrauchten Kalorien durch Integrieren einer Differenz zwischen der berechneten zweiten und regulären Kalorienverbrauchsrate über einen steten Teil der Trainingsperiode, während dessen die Herzfrequenz konstant ist, und nach einer Aufwärmzeit, während der die Herzfrequenz nicht konstant ist.

29. Verfahren nach Anspruch 28, wobei eine Änderungsrate der Kalorienverbrauchsrate des steten Teils nach der Aufwärmzeit null ist.

30. Verfahren nach Anspruch 29, wobei das Berechnen der verbrauchten Kalorien ferner Folgendes beinhaltet:

Berechnen einer ersten Kalorienverbrauchsrate anhand der erfassten ersten Herzfrequenz, und
Ermitteln einer Aufwärmtrainingskomponente durch Integrieren einer Differenz zwischen der berechneten ersten Kalorienverbrauchsrate und der berechneten zweiten Kalorienverbrauchsrate über die Aufwärmzeit innerhalb der Trainingsperiode, während der die Herzfrequenz nicht konstant ist.

31. Verfahren nach Anspruch 30, wobei das Berechnen der verbrauchten Kalorien ferner das Berechnen einer regulären Komponente durch Integrieren einer Differenz zwischen der berechneten ersten und regulären Verbrauchsraten über die Trainingsperiode beinhaltet.

32. Verfahren nach Anspruch 31, wobei die Aufwärmzeit zehn Prozent der Trainingsperiode ausmacht und der zweite Punkt der Trainingsperiode neunzig Prozent der Trainingsperiode entspricht.

33. Verfahren nach Anspruch 32, wobei das Berechnen der verbrauchten Kalorien ferner Folgendes beinhaltet:

Ermitteln des Geschlechts des Benutzers;
wobei, wenn der Benutzer männlich ist:
die reguläre Komponente $T*Y_0$ ist,
die Aufwärmtrainingskomponente $(T/10)*(Y_1(2)-Y_1(1))*0,5$ ist,
die Trainingskomponente $(9T/10)*(Y_1(2)-Y_1(1))$ ist,
wobei:

$$Y_0 = 0,01808*(X(1)-A+20,25)+C,$$

$$Y_1(1) = B_m*(X(1)-A)+C+0,3645,$$

$$Y_1(2) = B_m*(X(2)-A)+C+0,3645,$$

X(1) die erfasste erste Herzfrequenz ist,
X(2) die erfasste zweite Herzfrequenz ist,
A die Herzfrequenz im Ruhezustand des Benutzers ist,
C eine Konstante in Bezug auf einen Koeffizienten für den Kaloriengrundumsatz pro Minute ist,
T die Trainingsperiode ist,

$$B_m = 0,0109*(LBM/H^2)-0,0023*(\%FAT)-0,0007*(age)-0,0211,$$

LBM der Prozentanteil der den Muskeln zugeordneten Körpermasse ist,
H die Größe ist und
Age das Alter des Benutzers ist, und
wobei, wenn der Benutzer weiblich ist:
die reguläre Komponente $T*Y_0$ ist,
die Aufwärmtrainingskomponente $(T/10)*(Y_1(2)-Y_1(1))*0,5$ ist,
die Trainingskomponente $(9T/10)*(Y_1(2)-Y_1(1))$ ist,
wobei:

$$Y_0 = 0,00895*(X(1)-A+20,25)+C,$$

$$Y_1(1) = B_f*(X(1)-A)+C+0,1812,$$

$$Y_1(2) = B_f*(X(2)-A)+C+0,1812,$$

X(1) die erfasste erste Herzfrequenz ist,
X(2) die erfasste zweite Herzfrequenz ist,

A die Herzfrequenz im Ruhezustand des Benutzers ist,

C eine Konstante in Bezug auf einen Koeffizienten für den Kaloriengrundumsatz pro Minute ist,

T die Trainingsperiode ist,

$$B_f = 0,0140 * (LBM/H^2) - 0,0012 * (\%FAT) - 0,1254,$$

LBM der Prozentanteil der den Muskeln zugeordneten Körpermasse ist,

H die Größe ist und

Age das Alter des Benutzers ist.

34. Verfahren nach einem der Ansprüche 21 bis 33, wobei die erste Herzfrequenz und die zweite Herzfrequenz mit einem von einem Finger des Benutzers gemessenen Photoplethysografiesignal erfasst wird.

35. Computerprogramm, das einen Computerprogrammcode umfasst, der die Aufgabe hat zu bewirken, dass das Messgerät nach einem der Ansprüche 1 bis 20 die Schritte nach einem der Ansprüche 21 bis 34 ausführt, wenn das genannte Programm auf der Steuerung des genannten Messgerätes abgearbeitet wird.

36. Computerprogramm nach Anspruch 35, ausgestaltet auf einem rechnerlesbaren Medium.

**Revendications**

1. Dispositif de mesure utilisé pour mesurer les calories consommées par un sujet utilisateur, comprenant :

   un capteur de pulsations (120) agencé pour détecter une première fréquence cardiaque de l'utilisateur à un premier point d'une période d'exercice et une seconde fréquence cardiaque de l'utilisateur à un second point de la période d'exercice autre que le premier point, le capteur de pulsations (120) ne détectant aucune fréquence cardiaque entre les premier et second points ; et
   un contrôleur agencé pour recevoir les première et seconde fréquences cardiaques détectées, et qui calcule les calories consommées entre les premier et second points de la période d'exercice en utilisant la seconde fréquence cardiaque détectée, une fréquence cardiaque au repos prédéterminée de l'utilisateur, et un ou plusieurs facteurs sélectionnés parmi : âge de l'utilisateur, sexe de l'utilisateur, poids de l'utilisateur, taille de l'utilisateur et pourcentage du poids corporel attribué à la masse musculaire de l'utilisateur ;
   **caractérisé en ce que** le contrôleur est agencé pour calculer les calories consommées entre les premier et second points de la période d'exercice, en utilisant de plus la première fréquence cardiaque détectée.

2. Dispositif de mesure selon la revendication 1, dans lequel le contrôleur est agencé de sorte à calculer différentes vitesses de variation de la fréquence cardiaque pendant la période d'exercice, sur la base des première et seconde fréquences cardiaques détectées.

3. Dispositif de mesure selon la revendications 2, dans lequel le contrôleur est agencé de sorte à calculer :

   une première composante consommation de calories ayant une première vitesse de variation pendant une première période de temps à un début de la période d'exercice et pendant laquelle la fréquence cardiaque de l'utilisateur passe de la première fréquence cardiaque détectée à la seconde fréquence cardiaque détectée, et une seconde composante consommation de calories ayant une seconde vitesse de variation pendant une seconde période de temps qui suit la première période de temps et pendant laquelle la fréquence cardiaque de l'utilisateur est sensiblement la seconde fréquence cardiaque détectée.

4. Dispositif de mesure selon la revendication 3, dans lequel la première période de temps égale dix pour cent de la période d'exercice et la seconde période de temps égale quatrevingt-dix pour cent de la période d'exercice.

5. Dispositif de mesure selon la revendication 1, dans lequel :

   le contrôleur est agencé de sorte à calculer un premier taux de consommation de calories sur la base de la première fréquence cardiaque détectée, et un second taux de consommation de calories sur la base de la seconde fréquence cardiaque détectée, et
le contrôleur est agencé de sorte à intégrer une différence entre les premier et second taux de consommation de calories calculés pendant une phase d'échauffement pendant laquelle la fréquence cardiaque n'est pas constante afin de calculer la consommation de calories pendant la phase d'échauffement de la période d'exercice.

**6.** Dispositif de mesure selon la revendication 5, dans lequel le contrôleur est agencé de sorte à calculer une vitesse de variation du taux de consommation de calories pendant la phase d'échauffement pour qu'il augmente à une vitesse constante.

**7.** Dispositif de mesure selon la revendication 6, dans lequel le contrôleur est agencé de sorte à calculer une vitesse de variation pour qu'elle constitue une différence entre les premier et second taux de consommation de calories calculés, divisée par la durée de l'échauffement.

**8.** Dispositif de mesure selon la revendication 1, dans lequel :

le contrôleur est agencé de sorte à calculer un second taux de consommation de calories sur la base de la seconde fréquence cardiaque détectée,
le contrôleur est agencé de sorte à calculer un taux régulier de consommation de calories sur la base de la fréquence cardiaque au repos prédéterminée, la fréquence cardiaque au repos prédéterminée étant une fréquence cardiaque sensiblement présente au moment du réveil initial de l'utilisateur après un sommeil, et
le contrôleur est agencé de sorte à intégrer une différence entre le second taux de consommation calculé et le taux de consommation de calories normal pendant une partie stable de la période d'exercice pendant laquelle la fréquence cardiaque est constante et après une phase d'échauffement pendant laquelle la fréquence cardiaque n'est pas constante.

**9.** Dispositif de mesure selon la revendication 8, dans lequel une vitesse de variation du taux de consommation de calories pendant la partie stable égale zéro.

**10.** Dispositif de mesure selon la revendication 9, dans laquelle le contrôleur est agencé pour intégrer de plus une différence entre un premier taux de consommation de calories et le second taux de consommation de calories pendant la phase d'échauffement de la période d'exercice pendant laquelle la fréquence cardiaque n'est pas constante, et
le contrôleur est agencé de sorte à calculer le premier taux de consommation de calories sur la base de la première fréquence cardiaque détectée.

**11.** Dispositif de mesure selon la revendication 10, dans lequel :

le contrôleur est en outre agencé pour intégrer une différence entre le premier taux de consommation de calories et le taux de consommation de calories normal pendant la période d'exercice.

**12.** Dispositif de mesure selon la revendication 11, dans lequel la phase d'échauffement correspond à dix pour cent de la période d'exercice et le second point de la période d'exercice correspond à quatrevingt-dix pour cent de la période d'exercice.

**13.** Dispositif de mesure selon la revendication 12, dans lequel :

la composante normale est $T*Y_0$,
la composante phase d'échauffement est $(T/10)*(Y_1(2)-Y_1(1))*0.5$,
la composante exercice est $(9T/10)*(Y_1(2)-Y_1(1))$, expressions dans lesquelles :

$$Y_0 = 0,01808*(X(1)-A+20,25)+C,$$

$$Y_1(1) = B_m*(X(1)-A)+C+0,3645,$$

$$Y_1(2) = B_m*(X(2)-A)+C+0,3645,$$

X(1) est la première fréquence cardiaque détectée,

X(2) est la seconde fréquence cardiaque détectée,

A est la fréquence cardiaque au repos de l'utilisateur,

C est une constante relative à un coefficient de vitesse du métabolisme basal/minute,

T est la période d'exercice,

$$B_m = 0,0109*(LBM/H^2)-0,0023*(\% \text{ graisses corporelles})-$$
$$0,0007*(\text{âge})-0,0211,$$

LBM est le pourcentage de la masse corporelle attribué à la masse musculaire,

H est la taille, et

âge est l'âge de l'utilisateur.

**14.** Dispositif de mesure selon la revendication 11, dans lequel :

la composante normale est $T*Y_0$

la composante phase d'échauffement est $(T/10)*(Y_1(2)-Y_1(1))*0,5$,

la composante exercice est $(9T/10)*(Y_1(2)-Y_1(1))$, expressions dans lesquelles :

$$Y_0 = 0,00895*(X(1)-A+20,25)+C,$$

$$Y_1(1) = B_f*(X(1)-A)+C+0,1812$$

$$Y_1(2) = B_f*(X(2)-A)+C+0,1812$$

X(1) est la première fréquence cardiaque détectée,

X(2) est la seconde fréquence cardiaque détectée,

A est la fréquence cardiaque au repos de l'utilisateur,

C est une constante relative à un coefficient de vitesse du métabolisme basal/minute

T est la période d'exercice,

$$B_f = 0,0140(LBM/H^2)-0,0012(\% \text{ graisses corporelles})-0,1254,$$

LBM est le pourcentage de la masse corporelle attribué à la
masse musculaire,

H est la taille, et

âge est l'âge de l'utilisateur.

**15.** Dispositif de mesure selon l'une quelconque des revendications précédentes, dans lequel le contrôleur est au minimum un processeur, ledit processeur étant agencé de sorte à reproduire des données audio et/ou vidéo stockées dans une mémoire connectée au dispositif de mesure.

**16.** Dispositif de mesure selon la revendication 15, qui comprend de plus un boîtier (130) et une batterie à l'intérieur du boîtier (130), agencée de sorte à alimenter en énergie le au moins un processeur, le contrôleur étant disposé dans le boîtier (130), et le capteur de pulsations comprend une échancrure, prévue dans le boîtier (130), dimensionnée de sorte à recevoir le bout d'un doigt.

**17.** Dispositif de mesure selon l'une quelconque des revendications précédentes, dans lequel le premier point est à un début de la période d'exercice et le second point est à une fin de la période d'exercice.

**18.** Dispositif électrique portable en main (100) qui comprend le dispositif de mesure de l'une quelconque des revendications précédentes.

**19.** Le dispositif de mesure selon l'une quelconque des revendications 1 à 17, dans lequel :

à un premier point, le contrôleur signale à l'utilisateur qu'il doit prendre son pouls au moyen du capteur de pulsations (120) pour acquérir la première fréquence cardiaque et, au second point, le contrôleur signale à l'utilisateur qu'il doit prendre un autre pouls au moyen du capteur de pulsations (120) pour acquérir la seconde fréquence cardiaque, et

pendant au moins une partie de la période d'exercice autre qu'au premier et au second point, l'utilisateur ne prend aucun pouls au moyen du capteur de pulsations (120).

**20.** Dispositif de mesure selon l'une quelconque des revendications 1 à 17, dans lequel le capteur de pulsations (120) est agencé de sorte à détecter la première et la seconde fréquences cardiaques au moyen d'un signal photopléthysmographique mesuré à partir d'un doigt de l'utilisateur.

**21.** Méthode de mesure de la consommation de calories d'un utilisateur, la méthode comprenant :

détection d'une première fréquence cardiaque de l'utilisateur (520) à un premier point d'une période d'exercice, et d'une seconde fréquence cardiaque de l'utilisateur (560) à un second point de la période d'exercice, autre que le premier point ; et

calcul des calories consommées pendant la période d'exercice, sur la base de la première fréquence cardiaque détectée, de la seconde fréquence cardiaque détectée, d'une fréquence cardiaque au repos prédéterminée de l'utilisateur, et d'un ou plusieurs facteurs sélectionnés parmi : âge de l'utilisateur, sexe de l'utilisateur, poids de l'utilisateur, taille de l'utilisateur et le pourcentage de la masse corporelle attribué à la masse musculaire de l'utilisateur,

**caractérisée en ce que** le calcul des calories consommées entre les premier et second points pendant la période d'exercice utilise de plus la première fréquence cardiaque détectée.

**22.** Méthode selon la revendication 21, selon laquelle :

le calcul des calories consommées comprend la détermination d'une composante exercice qui correspond aux calories consommées pendant une période d'exercice, et correspondent à des calories consommées pendant une période d'exercice, et

la composante exercice a une vitesse de variation discontinue pour la fréquence cardiaque comprise entre les première et seconde périodes sur la base des première et seconde fréquences cardiaques détectées.

**23.** Méthode selon la revendication 22, selon laquelle la détermination de la composante exercice consiste à :

déterminer une première composante consommation de calories pendant une première période de temps au début de la période d'exercice et pendant laquelle la fréquence cardiaque de l'utilisateur passe de la première fréquence cardiaque détectée à la seconde fréquence cardiaque détectée, et

déterminer une seconde composante consommation de calories pendant une seconde période de temps après la première période pendant laquelle la fréquence cardiaque de l'utilisateur est sensiblement la seconde fréquence cardiaque détectée.

**24.** Méthode selon la revendication 23, selon laquelle la première période de temps correspond à dix pour cent de la période d'exercice et la seconde période de temps correspond à quatrevingt-dix pour cent de la période d'exercice.

**25.** Méthode selon la revendication 21, selon laquelle le calcul des calories consommées consiste à :

calculer un premier taux de consommation de calories sur la base de la première fréquence cardiaque détectée, calculer un second taux de consommation de calories sur la base de la seconde fréquence cardiaque détectée, et déterminer une composante phase d'échauffement en intégrant une différence entre les premier et second taux de consommation de calories pendant une phase d'échauffement comprise dans la période d'exercice lorsque la fréquence cardiaque n'est pas constante.

**26.** Méthode selon la revendication 25, selon laquelle la détermination de la composante phase d'échauffement comprend le calcul d'une vitesse de variation du taux de consommation de calories pendant la phase d'échauffement pour obtenir une augmentation constante.

**27.** Méthode selon la revendication 26, selon laquelle la vitesse de variation est une différence entre les premier et second taux de consommation de calories, divisée par la phase d'échauffement.

**28.** Méthode selon la revendication 21, selon laquelle le calcul des calories consommées consiste à :

calculer un second taux de consommation de calories sur la base de la seconde fréquence cardiaque détectée, calculer un taux de consommation de calories normal sur la base de la fréquence cardiaque au repos prédéterminée, mesurée lorsque l'utilisateur est sensiblement au moment d'un réveil initial après son sommeil, et déterminer une composante exercice des calories consommées en intégrant une différence entre le second taux de consommation de calories calculé et le taux de consommation de calories normal pendant une partie stable de la période d'exercice, pendant laquelle la fréquence cardiaque est constante et après une phase d'échauffement pendant laquelle la fréquence cardiaque n'est pas constante.

**29.** Méthode selon la revendication 28, selon laquelle une vitesse de variation du taux de consommation de calories de la partie stable après la phase d'échauffement égale zéro.

**30.** Méthode selon la revendication 29, selon laquelle le calcul des calories consommées comprend de plus :

le calcul d'un premier taux de consommation de calories sur la base de la première fréquence cardiaque détectée, et
la détermination d'une composante exercice d'échauffement en intégrant une différence entre le premier taux de consommation de calories calculé et le second taux de consommation de calories calculé pendant la phase d'échauffement de la période d'exercice pendant laquelle la fréquence cardiaque n'est pas constante.

**31.** Méthode selon la revendication 30, selon laquelle le calcul des calories consommées comprend de plus le calcul d'une composante normale en intégrant une différence entre le premier taux de consommation calculé et le taux de consommation normal pendant la période d'exercice.

**32.** Méthode selon la revendication 31, selon laquelle la phase d'échauffement correspond à dix pour cent de la période d'exercice et le second point de la période d'exercice correspond à quatrevingt-dix pour cent de la période d'exercice.

**33.** Méthode selon la revendication 32, selon laquelle le calcul des calories consommées comprend de plus :

la détermination du sexe de l'utilisateur/l'utilisatrice :

s'il s'agit d'un utilisateur masculin :

la composante normale est $T*Y_0$
la composante phase d'échauffement est $(T/10)*(Y_1(2)-Y_1(1))*0,5$,
la composante exercice est $(9T/10)*(Y_1(2)-Y_1(1))$, expressions dans lesquelles :

$$Y_0 = 0,01808*(X(1)-A+20,25)+C$$

$$Y_1(1) = B_m*(X(1)-A+C+0,3645,$$

$$Y_1(2) = B_m*(X+(2)-A)+C+0,3645,$$

X(1) est la première fréquence cardiaque détectée,
X(2) est la seconde fréquence cardiaque détectée,
A est la fréquence cardiaque de l'utilisateur au repos,
C est une constante relative à un coefficient de vitesse du métabolisme basal/minute,

T est la période d'exercice,

$$B_m = 0,0109*(LBM/H^2)-0,0023*(\% \text{ graisses corporelles})-0,0007*(\text{âge})-0,0211,$$

LBM est le pourcentage de la masse corporelle attribué à la masse musculaire,
H est la taille, et
âge est l'âge de l'utilisateur ; et
si le sujet utilisateur est féminin :

la composante normale est $T*Y_0$,

la composante phase d'échauffement est $(T/10)*(Y_1(2)-(Y_1(1))*0,5$,
la composante exercice est $(9T/10)*(Y_1(2)-Y_1(1))$, expressions dans lesquelles :

$$Y_0 = 0,00895*(X(1)-A+20,25)+C,$$

$$Y_1(1) = B_f*(X(1)-A)+C+0,1812,$$

$$Y_1(2) = B_f*(X(2)-A)+C+0,1812,$$

X(1) est la première fréquence cardiaque détectée,
X(2) est la seconde fréquence cardiaque détectée,
A est la fréquence cardiaque de l'utilisatrice au repos,
C est une constante relative à un coefficient de vitesse du métabolisme basal/minute,
T est la période d'exercice,

$$B_f = 0,0140(LBM/H^2)-0,0012(\% \text{ graisses corporelles})-0,1254,$$

LBM est le pourcentage de la masse corporelle attribué à la masse musculaire,
H est la taille, et
âge est l'âge de l'utilisatrice.

34. Méthode selon l'une quelconque des revendications 21 à 23, selon laquelle la première fréquence cardiaque et la seconde fréquence cardiaque sont détectées au moyen d'un signal photopléthysmographique mesuré à partir d'un doigt de l'utilisateur.

35. Programme informatique comprenant un code de programme informatique adapté pour faire exécuter, par le dispositif de mesure selon l'une quelconque des revendications 1 à 20, les étapes de l'une quelconque des revendications 21 à 34 lorsque ledit programme tourne sur le contrôleur dudit dispositif de mesure.

36. Le programme informatique selon la revendication 35, enregistré sur un support lisible par machine.

120    100

130

110

140

FIG. 1

DISPLAY — 210

CALORIE
MEASURING
DEVICE

100

COMPUTER — 200

FIG. 2

| Sports Fitness Manager | | | | | ⑦⊖⑩⊗ |

User ID : user01  Log out  Connecting...
Name : user01

Calorie List | Calorie Chart | Bio Rhythm | Profile

| Date | Exercising Time | Before HR | After HR | Consumed Calorie | |
|---|---|---|---|---|---|
| 06-10-2004 14:42 | 00:25 | 76.0 | 159.0 | 145 | |
| 06-10-2004 14:18 | 00:22 | 75.0 | 156.0 | 127 | |
| 06-10-2004 14:16 | 00:00 | 90.0 | 99.0 | 0 | |
| 06-10-2004 11:18 | 00:34 | 71.0 | 153.0 | 197 | |
| 06-10-2004 11:05 | 00:10 | 71.0 | 175.0 | 75 | |
| 06-10-2004 10:21 | 00:38 | 71.0 | 152.0 | 214 | |
| 06-10-2004 9:58 | 00:16 | 73.0 | 180.0 | 116 | |
| 06-09-2004 16:18 | 00:40 | 72.0 | 157.0 | 237 | |
| 06-09-2004 14:46 | 00:22 | 80.0 | 170.0 | 137 | |
| 06-09-2004 13:40 | 01:05 | 81.0 | 176.0 | 416 | |
| 06-09-2004 9:43 | 00:24 | 74.0 | 120.0 | 90 | |
| 04-23-2004 16:47 | 00:39 | 66.0 | 138.0 | 144 | |
| | | | | | |
| | | | | | |
| | | | | | |

< | >

# FIG. 3

```
┌─────────────────────────────┐
│    CALORIE MEASUREMENT      │──── 410
└─────────────────────────────┘
              │
              ▼
┌─────────────────────────────┐
│       PROFILE INPUT         │
│     NAME, GENDER, AGE,      │──── 420
│       WEIGHT, HEIGHT        │
└─────────────────────────────┘
              │
              ▼
┌─────────────────────────────┐
│   INITIAL HEART RATE CHECK  │──── 430
└─────────────────────────────┘
              │
              ▼
          440
        ◇
     ARE THE FINGERS
     ON THE SENSORS          YES        ┌─────────────────────┐
     AND THE START BUTTON ───────────── │   MEASURE CALORIE   │
       IS PRESSED?                      └─────────────────────┘
        ◇                                        450
        │
        NO
        │
        ▼
┌─────────────────────────────┐
│            END              │──── 460
└─────────────────────────────┘
```

FIG. 4

```
┌─────────────────────────┐
│    DETECTING SIGNAL     │──510
└─────────────────────────┘
            │
            ▼
┌─────────────────────────┐
│ HEART RATE MEASUREMENT  │──520
│    BEFORE EXERCISE      │
└─────────────────────────┘
            │
            ▼
┌─────────────────────────┐
│     END EXERCISE (T)    │──530
└─────────────────────────┘
            │
            ▼
        ╱ ARE THE FINGERS ╲
   NO  ╱  ON THE SENSORS    ╲
◄──────  AND THE START BUTTON  ──540
        ╲   IS PRESSED?     ╱
          ╲               ╱
                │ YES
                ▼
┌─────────────────────────┐
│    DETECTING SIGNAL     │──550
└─────────────────────────┘
                │
                ▼
┌─────────────────────────┐
│ HEART RATE AFTER EXERCISE│──560
└─────────────────────────┘
                │
                ▼
┌─────────────────────────┐
│    DISPLAY CALORIE      │──570
│  CONSUMPTION RESULTS    │
└─────────────────────────┘

  580
┌─────────────────────────┐
│          END            │
└─────────────────────────┘
```

# FIG. 5

FIG. 6

FIG. 7
PRIOR ART

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2080029 A
- US 20020019585 A1
- JP 8052119 A

### Non-patent literature cited in the description

- *Public Welfare Annuity Hospital Annual Report,* 1990, vol. 17, 201-206
- *Company Height Medical Science, Hirosaki Medical Department Report,* 1988, vol. 40 (1), 60-69